(19) Europäisches Patentamt
European Patent Office
Office européen des brevets

(11) Veröffentlichungsnummer: **0 320 437**
**A2**

# (12) EUROPÄISCHE PATENTANMELDUNG

(21) Anmeldenummer: **88730273.5**

(22) Anmeldetag: **05.12.88**

(51) Int. Cl.4: **A 61 K 31/56**
A 61 K 31/58

(30) Priorität: **07.12.87 DE 3741801**

(43) Veröffentlichungstag der Anmeldung:
**14.06.89 Patentblatt 89/24**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Jungblut, Peter, Prof. Dr.**
**Kampweg 4**
**D-3057 Neustadt-Büren (DE)**

**Wiechert, Rudolf, Prof. Dr.**
**Petzower Strasse 8A**
**D-1000 Berlin 39 (DE)**

**Bittler, Dieter**
**Bölkauer Pfad 11**
**D-1000 Berlin 27 (DE)**

Patentansprüche für folgende Vertragsstaaten: ES + GR.
Die Bezeichnung der Erfindung wurde geändert (Richtlinien für die Prüfung im EPA, A-III, 7.3).

## (54) 17-Methylen- und 17-Ethyliden-Estratriene mit östrogener Wirkung.

(57) Pharmazeutische Zubereitung zur Behandlung klimakterischer Beschwerden, zur Behandlung von hormonabhängigen Tumoren und zur Verwendung als Kontrazeptivum, gekennzeichnet durch den Gehalt an einer oder mehreren Verbindungen der allgemeinen Formel I

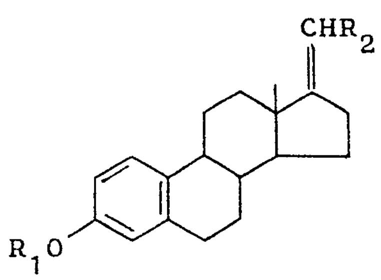

(I),

worin
$R_1$ ein Wasserstoffatom, eine Methyl-, Tetrahydropyranyl-oder Acylgruppe und
$R_2$ ein Wasserstoffatom oder eine Methylgruppe bedeuten.

**EP 0 320 437 A2**

## Beschreibung

### 17-Methylen- und 17-Ethyliden-estratriene

Die Erfindung betrifft neue pharmazeutische Zubereitungen und ihre Verwendung zur Behandlung klimakterischer Beschwerden, zur Behandlung von hormonabhängigen Tumoren und zur Verwendung als Kontrazeptivum.

Die pharmazeutische Zubereitung ist gekennzeichnet durch den Gehalt an einer oder mehreren Verbindung(en) der allgemeinen Formel I

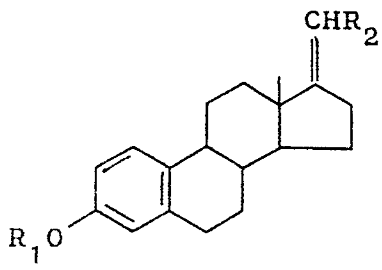

(I),

worin
$R_1$ ein Wasserstoffatom, eine Methyl-, Tetrahydropyranyl-oder Acylgruppe und
$R_2$ ein Wasserstoffatom oder eine Methylgruppe
bedeuten.

Als Acylgruppen kommen physiologisch verträgliche Gruppen infrage, die sich von Säuren ableiten, die üblicherweise zur Veresterung von Hydroxysteroiden verwendet werden. Hierzu zählen insbesondere organische Carbonsäuren mit 1 bis 12 Kohlenstoffatomen, die der aliphatischen, cycloaliphatischen, aromatischen, aromatisch-aliphatischen oder heterocyclischen Reihe angehören und die gesättigt oder ungesättigt, ein- oder mehrbasisch und/oder substituiert sein können. Als Beispiele für die Substituenten seien Alkyl-, Hydroxy-, Alkoxy-, Oxo- oder Aminogruppen und Halogenatome erwähnt. Hierzu zählen auch die gebräuchlichen anorganischen Säuren.

Als Carbonsäuren mit 1 bis 12 Kohlenstoffatomen seien beispielsweise genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Trimethylessigsäure, tert. Butylessigsäure, Cyclopentylessigsäure, Diäthylaminoessigsäure, Morpholinoessigsäure, Milchsäure, Bernsteinsäure, Adipinsäure, Benzoesäure, Nikotinsäure u.a.

Als anorganische Säuren kommen zum Beispiel Schwefel- und Phosphorsäure in Betracht.

Die Ester der Bernsteinsäure, Adipinsäure, Schwefelsäure und Phosphorsäure können gegebenenfalls mit Alkali in die wasserlöslichen Salze überführt werden.

Die Verbindungen der allgemeinen Formel I mit $R_1$ in der Bedeutung von Wasserstoff oder Acetyl sind bereits bekannt.

17-Methylen-1,3,5(10)-estratrien-3-ol und das 3-Acetat werden zum Beispiel in J. Org. Chem. 1981, 46, 3326-3328, als Zwischenprodukte zur Herstellung von D-Homosteroiden beschrieben. 17-Ethyliden-1,3,5(10)-estratrien-3-ol ist ein Zwischenprodukt zur Herstellung der entsprechenden 17β-Ethylverbindung (US-Patent 3 946 052). Von den 17-Methylenverbindungen heißt es in Chem. Abstracts Vol. 75 (1971) 151978z, daß diese Verbindungen Anticholesterinwirkung zeigen.

Es wurde nun gefunden, daß die in 17-Stellung durch Methylen oder Ethyliden substituierten Estratriene sich deutlich von den Estronen unterscheiden. Im Vergleich zu den Estronen, aus denen sie hergestellt werden, zeigen die Verbindungen der allgemeinen Formel I eine geringere Affinität zu den Östrogenrezeptoren als Estradiol und bewirken eine im Vergleich mit Estradiol erhöhte Zellmembran- und Blut-/Lymphgefäßpermeabilität.

Im Östrogen-Rezeptor-Bindungstest auf östrogene Wirkung unter Verwendung von Zytosol aus Schweineuterushomogenat und von 6,7$^3$H-Estradiol als Bezugssubstanz zeigen die Verbindungen der allgemeinen Formel I eine geringe Affinität zum Östrogenrezeptor.

In der folgenden Tabelle wird die Kompetition am Rezeptor in Prozent angegeben.

Tabelle

Östrogen-Rezeptor-Bindungstest

| Verbindung | Mol | % Kompetition |
|---|---|---|
| Estradiol | $2 \times 10^{-8}$ | 49 |
| | $2 \times 10^{-7}$ | 88 |
| | $2 \times 10^{-6}$ | 96 |
| 17-Methylen- | $2 \times 10^{-8}$ | 8 |
| 1,3,5(10)-estr- | $2 \times 10^{-7}$ | 25 |
| atrien-3-ol | $2 \times 10^{-6}$ | 73 |
| 17-Ethyliden- | $2 \times 10^{-8}$ | 5 |
| 1,3,5(10)-estr- | $2 \times 10^{-7}$ | 11 |
| atrien-3-ol | $2 \times 10^{-6}$ | 48 |

Im Uteruswachstumstest mit infantilen, 23 Tage alten Spraque-Dawley Ratten hat zum Beispiel 17-Methylen-1,3,5(10)-estratrien-3-ol nur 1/70 der uterotropen Aktivität von Estradiol. Der Wert von 1/70 der Estradiolwirkung wird sowohl gefunden, wenn man die Uterusfeuchtgewichte einschließlich der Intrauterinflüssigkeit zugrunde legt, als auch, wenn der DNA-Gehalt als Maß des Uteruszellgehalts genommen wird.

Zur Ausführung des Tests erhalten die infantilen weiblichen Ratten einmal täglich über 3 Tage subcutan Estradiol oder 17-Methylen-1,3,5(10)-estratrien-3-ol. Am 4. Tag werden die Tiere getötet und das Uterusgewicht oder der DNA-Gehalt pro Uterus bestimmt.

Ein Uterusgewicht von 50 mg oder ein DNA-Gehalt von 302 µg werden mit 0,07 µg Estradiol oder mit 5 µg 17-Methylen-1,3,5(10)-estratrien-3-ol erzielt.

Nach lokaler Applikation von Estradiol oder 17-Methylen-1,3,5(10)-estratrien-3-ol in das Uteruslumen des Schweins wird ein Uterusödem erzeugt, das bei der 17-Methylenverbindung etwa 30 Minuten später einsetzt als bei Estradiol, aber im gleichen Ausmaß auftritt wie bei Estradiol. Das Ausmaß des Ödems kann durch Bestimmung des Albumin- und des DNA-Gehalts ermittelt werden.

Die intrauterine Injektion von $1 \times 10^{-6}$ molaren Lösungen (20-50 ml/Uterus) der zu testenden Verbindungen führt bei Estradiol nach 120 Minuten zu einem Anstieg des Albumingehalts auf ca. 17 mg Albumin/1 mg DNA und bei der entsprechenden 17-Methylenverbindung nach 150 Minuten zu einem Anstieg auf den gleichen Wert.

Das Einbringen der Testsubstanz in ein Uterushorn des Schweins bewirkt nur dort eine Ödembildung; das unbehandelte Horn wird nicht beeinflußt.

Für die Verbindungen der allgemeinen Formel I findet man demnach eine Wirkungsdisproportionierung im Sinne einer geringeren Wirkung im Zellkern über den Östrogenrezeptor bei im Vergleich zum Estradiol unveränderter Ödembildung.

Als Substrate für intrazelluläre Enzyme, deren Produkte zu einer Erhöhung der Zellmembran- und Blut-/Lymphgefäßpermeabilität führen, die sich als sogenannte "Wasserimbibition" in Form eines massiven Ödems im Zielorgan Uterus demonstrieren läßt, sind die Verbindungen der allgemeinen Formel I besonders geeignet zur Behebung klimakterischer Beschwerden sowie allgemein zur Behandlung aller Erscheinungen, die durch Ausfall des zweiten Wirkungsabschnittes von Estradiol auftreten.

Die Wirkstoffe werden vorzugsweise oral appliziert, sie können jedoch auch lokal und parenteral verabreicht werden. Dazu werden die Wirkstoffe mit den in der galenischen Pharmazie üblichen Zusätzen, Trägerstoffen und/oder Lösungsvermittlern nach an sich bekannten Methoden zu den üblichen Applikationsformen verarbeitet. Für die bevorzugte orale Applikation kommen insbesondere Tabletten, Dragees, Kapseln, Pillen, wäßrige Suspensionen oder alkoholische Lösungen infrage und für die lokale und parenterale Applikation insbesondere Salben bzw. ölige Lösungen, wie zum Beispiel Sesam- oder Rizinusöllösungen, die gegebenenfalls zusätzlich einen Lösungsvermittler, wie zum Beispiel Benzylbenzoat, enthalten können.

Die Wirkstoffkonzentration in den pharmazeutischen Zubereitungen ist abhängig von der Applikationsform und vom Anwendungsgebiet. So können zum Beispiel Tabletten, Dragees, Kapseln oder Pillen 50 - 200 µg Wirkstoff pro Dosiseinheit und ölige Lösungen oder Salben 1 - 20 µg Wirkstoff pro ml enthalten.

In einer bevorzugten Ausführungsform enthält die orale Applikationsform 50 bis 150 µg Wirkstoff.

Nach einer Behandlung von therapeutisch kastrierten Frauen sowie von Frauen im Klimakterium, die jeweils unter Hitzewallungen und Niedergeschlagenheit litten, mit täglich 50 bis 150 µg Wirkstoff der allgemeinen Formel I trat bereits nach 2 Tagen eine deutliche Besserung der Beschwerden auf.

Die systemische Gabe von Verbindungen der allgemeinen Formel I an Spraque-Dawley Ratten mit durch 7,12-Dimethylbenzanthracen induzierten Mammatumoren führt zu einem Sistieren des Tumorwachstums ohne merkliche Beeinflussung des Zyklus. Die Verbindungen sind damit auch zur Behandlung von hormonabhängigen Tumoren geeignet. Durch Anwendung von täglich 0,15 - 15 µg per kg Wirkstoff wird die Wachstumsförderung unterbunden.

Die Antitumorwirkung kann wie in Science 137 (1962) 257-262 beschrieben näher bestimmt werden..

Als Substanzen mit selektiver östrogener Wirkung können die Verbindungen der allgemeinen Formel I auch in Präparaten zur Kontrazeption verwendet werden, vorzugsweise in Kombination mit einer gestagen

wirksamen Hormonkomponente, wie zum Beispiel Levonorgestrel, Gestoden oder Desogestrel. Oral applizierbare Applikationsformen enthalten vorzugsweise 50 - 200 µg einer Verbindung der allgemeinen Formel I und 50 - 500 µg eines stark wirksamen Gestagens.

Die Verbindungen der allgemeinen Formel I sind entweder bekannt oder können aus den bekannten 3-Hydroxyverbindungen nach an sich bekannten Methoden durch Veresterung oder Verätherung hergestellt werden.

Die Veresterung (Acylierung) erfolgt vorzugsweise mit Pyridin/Säureanhydrid oder Pyridin/Säurechlorid bei Raumtemperatur.

Zur Verätherung dienen alkylierende Verbindungen, wie Diazoalkane oder Dialkylsulfate.

**Vorschriten für die Herstellung von Verbindungen der allgemeinen Formel I**

**1.) 17-Methylen-1,35(10)-estratrien-3-ol**

Zu einer Suspension von 44.0 g Methyltriphenylphosphoniumbromid in 200 ml Dioxan werden unter dem Argonstrom und Eisbadkühlung 74,6 ml 15 %ige Butyllithiumlösung in Hexan zugetropft. Es wird dann 1,5 Stunden bei Raumtemperatur nachgerührt. Dann werden 6,0 g 3-(Tetrahydropyran-2′-yloxy)-1,3,5(10)-estratrien-17-on zugegeben, das Hexan bis zu einem Siedepunkt von 100 °C abdestilliert und anschließend die Reaktionslösung 19 Stunden refluxiert. Nach Eiswasserfällung wird die Substanz mit Dichlormethan extrahiert. Nach dem Trocknen und Eindampfen wird der Rückstand an Kieselgel chromatographiert, und es werden 6,1 g 17-Methylen-3-(tetrahydropyran-2′-yloxy)-1,3,5(10)-estratrien vom Schmelzpunkt 73,5 °C erhalten.

Zu einer Lösung von 6,0 g 17-Methylen-3-(tetrahydropyran-2′-yloxy)-1,3,5(10)-estratrien in 60 ml Methanol werden 6 ml 8 Vol.-%ige Schwefelsäure gegeben und 4 Stunden bei Raumtemperatur stehengelassen. Die Reaktionslösung wird dann mit Ether verdünnt, mit Wasser gewaschen, getrocknet und eingedampft. Der Rückstand wird aus Hexan umkristallisiert, und es werden 4,1 g 17-Methylen-1,3,5(10)-estratrien-3-ol vom Schmelzpunkt 133,5 °C erhalten.

**2.) 3-Methoxy-17-methylen-1,3,5(10)-estratrien**

200 mg 17-Methylen-1,3,5(10)-estratrien-3-ol werden in 115 ml 0,5 molarer Natronlauge gelöst, mit 2,5 ml Dimethylsulfat versetzt und über Nacht bei Raumtemperatur gerührt. Die Reaktionslösung wird mit Dichlormethan extrahiert, die organische Phase mit Wasser gewaschen, getrocknet und eingedampft. Der Rückstand wird an Kieselgel chromatographiert, und es werden 170 mg 3-Methoxy-17-methylen-1,3,5(10)-estratrien erhalten.

**3.) 3-Acetoxy-17-methylen-1,3,5(10)-estratrien**

200 mg 17-Methylen-1,3,5(10)-estratrien-3-ol werden in 1 ml Pyridin und 0,5 ml Acetanhydrid über Nacht bei Raumtemperatur stehengelassen. Es wird dann mit Eiswasser verdünnt, der ausgefallene Niederschlag abfiltriert, in Dichlormethan aufgenommen, getrocknet und eingedampft. Der Rückstand wird an Kieselgel chromatographiert, und es werden 185 mg 3-Acetoxy-17-methylen-1,3,5(10)-estratrien erhalten.

**4.) 3-Butyryloxy-17-methylen-1,3,5(10)-estratrien**

250 mg 17-Methylen-1,3,5(10)-estratrien-3-ol werden, wie im Beispiel 3 beschrieben, in 1,5 ml Pyridin mit 0,75 ml Buttersäureanhydrid umgesetzt und aufgearbeitet. Nach Chromatographie an Kieselgel werden 245 mg 3-Butyryloxy-17-methylen-1,3,5(10)-estratrien erhalten.

**5.) 17-Ethyliden-1,3,5(10)-estratrien-3-ol**

Zu einer Suspension von 10,6 g Ethyltriphenylphosphoniumbromid in 50 ml Dimethylsulfoxid werden portionsweise 3,22 g Kalium-tert.-butylat gegeben und 1 Stunde bei Raumtemperatur nachgerührt. Dann wird eine Lösung von 4.0 g tert.-Butyl-dimethylsilyloxy-1,3,5(10)-estratrien-17-on in 50 ml Dimethylsulfoxid zugetropft und weitere 3 Stunden bei Raumtemperatur nachgerührt. Die Reaktionslösung wird mit Ether verdünnt, mit Wasser gewaschen, getrocknet und eingedampft. Der Rückstand wird an Kieselgel chromatographiert, und es werden 450 mg (syn) 17-Ethyliden-1,3,5)10)-estratrien-3-ol erhalten.

**6.) 17-Ethyliden-1,3,5(10)-estratrien-3-methylether**

100 mg (syn) 17-Ethyliden-1,3,5(10)-estratrien-3-ol werden in 57,5 ml 0,5 molarer Natronlauge mit 1,25 ml Dimethylsulfat, wie im Beispiel 2 beschrieben, umgesetzt und aufgearbeitet. Nach Chromatographie an Kieselgel werden 75 mg (syn) 17-Ethyliden-1,3,5(10)-estratrien-3-methylether erhalten.

**7.) 17-Ethyiliden-3-propionyloxy-1,3,5(10)-estratrien**

100 mg (syn) 17-Ethyliden-1,3,5(10)-estratrien-3-ol werden in 1 ml Pyridin mit 0,5 ml Propionsäureanhydrid, wie im Beispiel 3 beschrieben, umgesetzt und aufgearbeitet. Nach Chromatographie an Kieselgel werden 95 mg (syn) 17-Ethyliden-3-propionyloxy-1,3,5(10)-estratrien erhalten.

<u>Beispiel 1</u>

**Zusammensetzung eines Dragees**

| | |
|---|---|
| 0,050 mg | 17-Methylen-1,3,5(10)-estratrien-3-ol |
| 46,450 mg | Lactose |
| 26,800 mg | Maisstärke |
| 3,000 mg | Poly(1-vinyl-2-pyrrolidon) Mittleres MG 25 000 |
| 3,700 mg | Talkum |
| 80,000 mg | Gesamtgewicht, das mit üblicher Zuckermischung auf etwa 140 mg ergänzt wird. |

Beispiel 2

**Zusammensetzung einer alkoholischen Lösung**

1 mg 17-Methylen-1,3,5(10)-estratrien-3-ol werden in 10 ml 46 %igen Ethylalkohol gelöst. In zehn Tropfen (0,5 ml) sind 50 µg Wirkstoff enthalten.

**Patentansprüche**

1.) Pharmazeutische Zubereitung zur Behandlung klimakterischer Beschwerden, zur Behandlung von hormonabhängigen Tumoren und zur Verwendung als Kontrazeptivum, gekennzeichnet durch den Gehalt an einer oder mehreren Verbindungen der allgemeinen Formel I

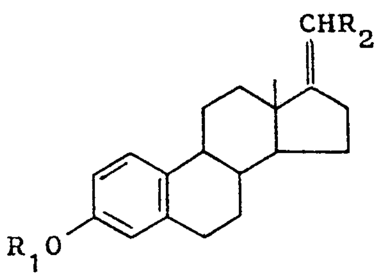

(I),

worin
$R_1$ ein Wasserstoffatom, eine Methyl-, Tetrahydropyranyl-oder Acylgruppe und
$R_2$ ein Wasserstoffatom oder eine Methylgruppe
bedeuten.

2.) Verwendung von Verbindungen der allgemeinen Formel I zur Herstellung einer pharmazeutischen Zubereitung zur Behandlung klimakterischer Beschwerden, zur Behandlung von hormonabhängigen Tumoren und zur Verwendung als Kontrazeptivum.

3.) Verfahren zur Herstellung einer pharmazeutischen Zubereitung mit einem Gehalt an einer oder mehreren Verbindungen der allgemeinen Formel I, dadurch gekennzeichnet, daß man die Wirkstoffe mit einem oder mehreren inerten Zusatzstoff(en) mischt und in die gewünschte Applikationsform überführt.

**Patentanspruch für folgende Vertragsstaaten: GR, ES**

Verfahren zur Herstellung einer pharmazeutischen Zubereitung zur Behandlung klimakterischer Beschwerden von hormonabhängigen Tumoren und zur Verwendung als Kontrazeptivum, dadurch gekennzeichnet, daß man eine oder mehrere Verbindungen der allgemeinen Formel I

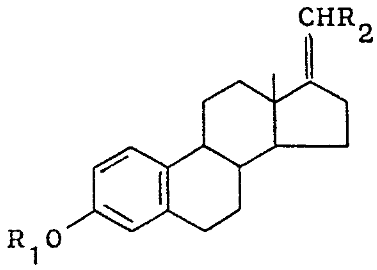

(I),

worin
$R_1$ ein Wasserstoffatom, eine Methyl-, Tetrahydropyranyl- oder Acylgruppe und
$R_2$ ein Wasserstoffatom oder eine Methylgruppe
bedeuten, mit einem oder mehreren inerten Zusatzstoff(en) mischt und in die gewünschte Applikationsform überführt.